# EUROPÄISCHE PATENTSCHRIFT

(11) **EP 2 190 982 B1**
(45) Veröffentlichungstag und Bekanntmachung des Hinweises auf die Patenterteilung: **17.04.2013**
(21) Anmeldenummer: 08785277.8
(22) Anmeldetag: 01.08.2008
(51) Int. Cl.: C12N 7/00

(54) **SYSTEM, UMFASSEND WIRKSTOFFE UMFASSENDE PARTIKEL UND BAKTERIOPHAGEN**
SYSTEM COMPRISING BACTERIOPHAGES AND PARTICLES THAT CONTAIN ACTIVE SUBSTANCES
SYSTÈME COMPRENANT DES PARTICULES CONTENANT DES PRINCIPES ACTIFS, AINSI QUE DES BACTÉRIOPHAGES

(30) Priorität: 06.08.2007 DE 102007036866
(43) Veröffentlichungstag der Anmeldung: 02.06.2010
(73) Patentinhaber: Bayer Intellectual Property GmbH, 40789 Monheim (DE)
(72) Erfinder: EIDEN, Stefanie, 51371 Leverkusen (DE); EBLE, Axel, 10405 Berlin (DE); WEISS, Martin, 53332 Bornheim/Sechtem (DE); DUFF, Daniel, Gordon, 51373 Leverkusen (DE); BORK, Olaf, 28816 Stuhr (DE); EGGER, Holger, 51067 Köln (DE); BUDDE, Bastian, 51063 Köln (DE); PLUG, Sascha, 51375 Leverkusen (DE)
(74) Vertreter: BIP Patents
(86) Internationale Anmeldenummer: PCT/EP2008/006336
(87) Internationale Veröffentlichungsnummer: WO 2009/018964

(56) Entgegenhaltungen:
- CHEN LIMOR ET AL: "Design and validation of a bifunctional ligand display system for receptor targeting." CHEMISTRY & BIOLOGY AUG 2004, Bd. 11, Nr. 8, August 2004 (2004-08), Seiten 1081-1091, XP002418492 ISSN: 1074-5521
- BROWN KATHLYNN C: "Hitting the target with bifunctional phage." CHEMISTRY & BIOLOGY AUG 2004, Bd. 11, Nr. 8, August 2004 (2004-08), Seiten 1033-1035, XP002504986 ISSN: 1074-5521
- LIGHT J ET AL: "PhoPhabs: Antibody-phage-alkaline phosphatase conjugates for one step ELISA's without immunization" BIOORGANIC & MEDICINAL CHEMISTRY LETTERS, PERGAMON, ELSEVIER SCIENCE, GB, Bd. 2, Nr. 9, 1. Januar 1992 (1992-01-01), Seiten 1073-1078, XP002420041 ISSN: 0960-894X
- NEWTON JESSICA R ET AL: "Melanoma imaging with pretargeted bivalent bacteriophage." JOURNAL OF NUCLEAR MEDICINE : OFFICIAL PUBLICATION, SOCIETY OF NUCLEAR MEDICINE MAR 2007, Bd. 48, Nr. 3, März 2007 (2007-03), Seiten 429-436, XP002504987 ISSN: 0161-5505

## Beschreibung

Die vorliegende Erfindung betrifft ein System, umfassend Wirkstoffe umfassende Partikel und Bakteriophagen. Diese Wirkstoff enthaltenden Partikel sind entweder reine Wirkstoffpartikel oder Kapseln, die einen Wirkstoff enthalten und werden im Folgenden kurz "Partikel" genannt. Erfindung betrifft weiterhin die Verwendung das Systems zur Fixierung von Wirkstoffen auf Substraten.

Bekannte Methoden zur Haftvermittlung von Wirkstoffen sind z.B. Imprägnierung, kovalente Bindung des Wirkstoffs auf die Oberfläche eines Substrats oder Integration des Wirkstoffs in einem Material und Verzögerung der Freisetzung des Wirkstoffs mittels Zusatzstoffen oder durch Integration von langzeitfreisetzenden Formulierungen wie z.B. Kapseln in dem Material. Eine Übersicht über Wirkstoffbindung an Substrate findet sich z.B. bei Kalász und Antal, Current Medicinal Chemistry, 2006, 13, 2535-2563.

Je nach Substrat erfordern die Imprägnierungsmethoden eine erhöhte und häufige Behandlung des Substrats, mit einem höheren Nebenwirkungsrisiko. Wirkstoffe oder Wirkstoffmischungen können durch Umwelteinflüsse bzw. Waschungen ausgewaschen werden, wenn die Haftung unzureichend ist. Eine kovalente chemische Bindung oder eine Integration von Partikeln kann außerdem je nach Substrat schlecht möglich (Haare, Haut) oder gesundheitsbedenklich (Textilien) sein.

Die Kopplung eines anorganischen Substrats mit biologischen Komponenten zur Modifikation der Oberflächeneigenschaften ist in der Biomimetik bekannt. Aus einer Phagenbibliothek selektierte, kurze Peptide präsentierende Bakteriophagen wurden bisher beispielsweise dazu verwendet, anorganische Materialien zu fällen bzw. abzuscheiden (WO2003/078451). Auch Hybridmaterialien aus einem anorganischen Substrat und spezifischen Polypeptid-Liganden werden als potentielle Lösung zur Veränderung der Substratoberfläche verwendet. Die Identifikation des an das Substrat passenden biologischen Liganden (üblicherweise ein Peptid) ist aber zeit- und kostspielig und stand bisher einer konkreten Anwendung im Wege. Das Konzept eines bifunktionellen Liganden zur Bindung von zwei anorganischen Komponenten wird im Stand der Technik angesprochen (siehe z. B. Sarikaya et al., Nature Materials, 2003, 2, 577-585). Konkret wurde die Bindung von Zellen oder Biomolekülen an einem Polymersubstrat, insbesondere an oxidiertem Chlor-angereicherten Polypyrrol (PPyC1) bzw. Poly(lactat-co-glycolat) (PLGA), durch Bakteriophagen mit bifunktionellen Bindungseigenschaften z.B. in W02004/035612 beschrieben. WO2004/035612 erwähnt die prinzipielle Möglichkeit, Arzneimittel mit dieser Methode an das Substrat zu binden, beschreibt aber ausschließlich die Identifikation des Polymer-spezifischen Phagen durch sogenanntes Biopanning und Bindung des Phagen an das Polymersubstrat. Die weitere Bindung an einen weiteren Stoff, der wie z. B. ein Arzneimittel kein biologischer Bindungspartner des verwendeten Phagen ist, wird nicht weiter beschrieben, wobei diese Bindung ebenfalls selektiv zu erfolgen hätte und eine genaue Anpassung des Liganden an die weitere Bindungskomponente erfordern würde.

Es bestand daher die Aufgabe, ein System, umfassend Wirkstoffe umfassende Partikel und Bakteriophagen bereitzustellen, wobei der Phage auf dem Partikel haftet und weiterhin zur Haftung auf einer Substratoberfläche geeignet ist.

Diese Aufgabe wurde erfindungsgemäß dadurch gelöst, dass auf Proteinen des Bakteriophagen ein erstes zusätzliches Peptid fusioniert ist, das erste zusätzliche Peptid an der Oberfläche des Partikels haftet und wobei weiterhin auf Proteinen des Bakteriophagen ein zweites zusätzliches Peptid fusioniert ist.

Wirkstoffe sind zunächst einmal Substanzen oder Substanzgemische, welche eine vom Anwender gewünschte Wirkung auf einen Organismus haben, gleich ob dieser menschlicher, pflanzlicher oder sonstiger Natur ist. Im Sprachgebrauch der vorliegenden Erfindung bezeichnen Proteine des Bakteriophagen Genprodukte des Phagengenoms, welche beispielsweise die Hülle des Bakteriophagen aufbauen. Ein erstes zusätzliches Peptid ist ein Peptid, welches nicht auf der natürlichen Form des Phagen enthalten ist, sondern beispielsweise mittels molekulargenetischer Manipulationen des Phagengenoms auf dem Phagen präsentiert wird. Das Peptid ist an Proteine des Bakteriophagen fusioniert, was bedeutet, dass es entweder N-terminal oder C-terminal über eine Peptidbindung mit dem Protein verbunden ist.

Wirkstoffe umfassende Partikel im Sinne der vorliegenden Erfindung sind insbesondere Mikropartikel, die einen Partikeldurchmesser mit einem d90-Wert der volumengewichteten Verteilung ≥0,1 bis ≤ 300, bevorzugt ≥ 0,3 bis ≤ 100, besonders bevorzugt ≥ 0,5 bis ≤ 30 Mikrometer aufweisen. Teilchen mit solchen Größen sind einerseits gut zum Anhaften an erfindungsgemäße Bakteriophagen geeignet. Andererseits sind Teilchen solcher Größen mit den oben beschriebenen Verfahren zur Herstellung von Mikropartikeln vorteilhaft. Der d90-Wert der volumengewichteten Verteilung ist diejenige Partikelgröße, für die gilt, dass 90% des Partikelvolumens von Partikeln kleiner oder gleich dem d90-Wert gebildet werden. Messmethoden zu Bestimmung der volumengewichteten Verteilung sind beispielsweise in Terence Allen: Particle Size Measurement, Kluwer Academic Publishers, Dordrecht/Boston/London 1999, S.404 ff aufgeführt. Die Darstellung der Ergebnisse ist ebenfalls in dieser Literaturquelle genannt oder kann auch nach der Norm DIN ISO 9276-1 erfolgen.

Das erste zusätzliche Peptid hat die Eigenschaft, dass es an der Oberfläche des Partikels haftet. Folglich sind Partikel und Bakteriophage über das erste Peptid miteinander verbunden. Die Art der Bindung kann beispielsweise eine kovalente Anbindung, eine elektrostatische Wechselwirkung über geladene oder teilgeladene funktionelle Gruppen oder eine Wassersto$brückenbindung sein. Ist ein Wirkstoff verkapselt, bindet eine der Komponenten der Kapsel an den Phagen. Liegt der Wirkstoff als reiner Wirkstoffpartikel vor, erfolgt die Bindung des Phagen direkt am reinen Wirkstoff. Beide Formen sind der vorliegenden Erfindung als Bindung zunächst unter der Bezeichnung "Partikel" zusammengefasst.

Das zweite zusätzliche Peptid ist ebenfalls ein Peptid, welches nicht auf der natürlichen Form des Phagen enthalten ist, sondern beispielsweise mittels molekulargenetischer Manipulationen des Phagengenoms auf dem Phagen präsentiert wird. Das Peptid ist an Proteine des Bakteriophagen fusioniert, was bedeutet, dass es entweder N-terminal oder C-terminal über eine Peptidbindung mit dem Protein verbunden ist. Dieses Peptid ist dazu bestimmt, die Haftung des Phagen und damit auch des Wirkstoffe umfassenden Partikels an einer Substratoberfläche zu ermöglichen. Die Art der Bindung kann dabei beispielsweise eine kovalente Anbindung, eine elektrostatische Wechselwirkung über geladene oder teilgeladene funktionelle Gruppen oder eine Wasserstoffbrückenbindung sein.

In einer weiteren Ausführungsform der vorliegenden Erfindung wurde die Aminosäuresequenz des zweiten zusätzlichen Peptids durch Panning einer kombinatorischen Phagenpopulation an einer Substratoberfläche ermittelt. Für die evolutive Selektion von einigen Phagenspezies aus einer großen kombinatorischen Phagenpopulation ("Phage-Display Bibliothek") an einem Substrat wird üblicherweise eine Phage-Display Bibliothek in gepufferter wässriger Umgebung einem Substrat ausgesetzt, so dass die Bindung von einigen Phagen stattfinden kann. Unspezifisch bindende und schwach bindende Phagen werden durch Benutzung eines wässrigen Waschpuffers abgewaschen. Nach dem Waschen noch bindende und damit spezifische Phagen werden anschließend durch Benutzung eines anderen wässrigen Puffers, später genannt Elutionspuffer, abgelöst (eluiert). Diese gesamte Prozedur wird "Panning" genannt. Die eluierten Phagen werden vermehrt und in weiteren Panning-Runden erneut dem Substrat ausgesetzt, bis sich eine Population von gut bindenden Phagen anreichert. Beispiele dieser Technik finden sich bei Sarikaya et al., Nature Materials, 2003, 2, 577-585; O'Neil und Hoess, Current Opinion in Structural Biology, 1995, 5, 443-449; Smith und Scott, Methods in Enzymology 1993, 217, 228-257; Sambrook und Russell (Hrsg), 2001, Molecular cloning: A laboratory manual (third edition), Cold Spring Harbor Press, Seite 18.115 bis 18.122. Die Aminosäuresequenz selbst, die für die Bindung verantwortlich ist, kann mittels gentechnischer oder molekularbiologischer Methoden ermittelt werden.

In einer weiteren Ausführungsform der vorliegenden Erfindung sind die Bakteriophagen vom Typ M13. Diese Phagen sind gut veränderbar und leicht erhältlich und in Kultur leicht vermehrbar. Sie weisen als Genprodukte beispielsweise die Proteine gpIII, gpV, gpVI, gpVII, gpVIII und gpIX auf. Es ist möglich, dass das erste zusätzliche Peptid an das Protein gpIII oder gpVIII fusioniert ist und das zweite zusätzliche Peptid an das Protein gpIII oder gpVIII fusioniert ist. Die Verlängerung dieser Proteine mit zusätzlichen Peptiden, also das Fusionieren, ist an diesen Proteinen gut durchführbar. Besonders bevorzugt ist, wenn das Protein gpIII N-terminal mit einem Peptid der Sequenz ISSKPTSQLTTP fusioniert ist und dass das Protein gpVIII N-terminal mit einem Peptid der Sequenz STTRLR fusioniert ist.

In einer weiteren Ausführungsform der vorliegenden Erfindung sind die Wirkstoffe ausgewählt aus der Gruppe umfassend Insektizide und/oder Fungizide, vorzugsweise Imidacloprid, Deltamethrin, Pemethrin, Clotrimazol, Bifonazol, Preventol und/oder Trifoxystrobin; sowie weiterhin Dexpanthenol. Solche Wirkstoffe profitieren besonders von der Möglichkeit, sie an eine Substratoberfläche anzubinden.

In einer weiteren Ausführungsform der vorliegenden Erfindung sind die Wirkstoffe umfassenden Partikel verkapselte Wirkstoffe. Es ist bei verschiedenen Anwendungsgebieten, beispielsweise bei der Anwendung von Agrochemikalien oder pharmazeutischen Wirkstoffen, wünschenswert, zur topischen Behandlung eine langfristige Anhaftung von Wirkstoffen und deren möglichst kontrollierter Freisetzung an beispielsweise Menschen oder Pflanzen zu erzielen. Hierzu werden an den zuvor beschriebenen gentechnisch veränderten bifunktionellen Bakteriophagen Mikropartikel angebunden, die eine Matrix beziehungsweise einen Kern, eine Hülle sowie einen oder mehrere Wirkstoffe umfassen.

Wirkstoffe enthaltende Partikel, insbesondere Mikropartikel, umfassen einen oder mehrere Wirkstoffe, die als Matrix- oder Kern-Hülle Partikel formuliert werden können. Zur Herstellung solcher Mikropartikel kommen Verfahren wie die Sprühtrocknung, Scheibenversprühung, Polymerisierungen (Grenzflächen, in situ etc.), Koazervation und Extrusion mit anschließender Mahlung zur Anwendung. Weitere Herstellverfahren sind in z.B. "Microcapsule Processing and Technology" (Asaji Kondo, ISBN 0824768574, Jahr 1979, 1ff) beschrieben.

Als Beispiele für solche Wirkstoffe wären Insektizide wie Imidacloprid, Deltamethrin oder Pemethrin sowie Fungizide wie Clotrimazol, Bifonazol, Preventol und Trifoxystrobin zu nennen.

"Matrix" beziehungsweise "Hülle" im Sinne der vorliegenden Erfindung bezeichnet die den Wirkstoff umgebende Substanz oder Substanzgemische. Die Matrix beziehungsweise Hülle kann zusätzlich zum Wirkstoff folgende Hilfsstoffe umfassen: Bindemittel, synthetische Makromoleküle, Füllstoffe, Dispergiermittel, Konservierungsmittel, Antioxidantien, Benetzungsmittel, Tenside und/oder Weichmacher beziehungsweise Plastifizierungsmittel.

Die Matrix ist dabei so gewartet, dass die zeitliche Freisetzung des Wirkstoffs an die jeweilige Aufgabenstellung angepasst ist.

Es ist möglich, dass das Gesamtgewicht der Matrix beziehungsweise Hülle, bezogen auf das Gesamtgewicht des Mikropartikels ≥ 1 Gew.-% bis ≤ 99,9 Gew.%, bevorzugt ≥ 5 Gew.-% bis ≤ 90 Gew.-%, mehr bevorzugt ≥ 10 Gew.-% bis ≤ 80 Gew.-% ausmacht. Solche Matrixbeziehungsweise Hüllanteile erlauben es, erfindungsgemäße Bakteriophagen an die verwendeten Hilfs- bzw. Wirkstoffe anzuhaften.

Weiterhin kann die Matrix Bindemittel, synthetische Makromoleküle und/oder Füllstoffe umfassen. Diese haben unter anderem die Funktion, die einzelnen Bestandteile des Mikropartikel formstabil zusammenzuhalten und/oder den Wirkstoff beziehungsweise Wirkstoffgemische kontrolliert freizusetzen.

Beispiele für Bindemittel können ausgewählt sein aus der Gruppe umfassend Cellulose-Derivate, mikrokristalline Cellulose, Natrium-Carboxymethylcellulose, Methylcellulose, Hydroxypropylcellulose, Hydroxypropylmethylmethylcellulose, Lactose, Stärke (Weizen-, Mais-, Kartoffel-, Reisstärke), Stärkederivate, Saccharose, Glucose, Mannit, Sorbit, Dicalciumphospat, Tricalciumphosphat, Bolus, Zinkoxid, Gelatine, Maltodextrine, Polysaccharide, Oligosaccharide, Stearinsäure, Calciumstearat, Schellack, Celluloseacetatphthalat und/oder Hydroxylmethylcellulosephthalat.

Beispiele für synthetische Makromoleküle können ausgewählt sein aus der Gruppe umfassend Copolymerisate von Dimethylaminoethacrylsäure und neutralen Methacrylsäureestern, Acryl- und Methacrylsäureester-Copolymerisate mit Trimethylammoniummethacrylat, Polymerisate von Methacrylsäure und Methacrylsäureestern, Acrylsäureäthylester-Methacrylsäuremethylester-Copolymerisat, Styrol, Acrylnitril Copolymer, Polystyrol, Polycarbonat, Polyester, Polyether, Polyamide, Polyimide, Polyharnstoffe, Polyurethane, Polysulfide, Polyvinylpyrrolidon, Polyvinylpyrrolidon-Vinylacetat und/oder Polyvinylalkohole.

Beispiele für Füllstoffe können ausgewählt sein aus der Gruppe umfassend Mineralien wie Tonmineralien und/oder kolloidale Kieselsäure wie zum Beispiel Kaolin, Kaolinit, Halloysit, Montmorillonit, Talkum, Bentonit, Vermiculit und/oder Allophan. Weiterhin umfassen Mineralien Oxide, Hydroxide, Silikate, Carbonate und Sulfate von Calcium, Magnesium, Aluminium und Titan.

Weiterhin kann die Matrix weitere Hilfsstoffe umfassen, die ausgewählt sind aus der Gruppe umfassend anionenaktive Tenside, nichtionogene Tenside und/oder Lipide mit Esterverbindung.

Beispiele für anionenaktive Tenside können ausgewählt sein aus der Gruppe umfassend Seifen, Salze von Fettsäuren, Natziumpalmitat, Natriumstearat, Natriumoleat, Natriumsalze von Fettalkoholsulfaten, Sulfoccinate, Alkylnaphtalinsulfonate, und/oder Alkylsulfate.

Beispiele für nichtionogene Tenside können ausgewählt sein aus der Gruppe umfassend partielle Fettsäureester mehrwertiger Alkohole, partielle Fettsäureester der Sorbitane, partielle Fettsäureester des Polyhydroxyethylensorbitans, Polyhydroxyethylen-Fettalkoholether, Polyhydroxyethylen-Fettsäure-ester, Ethylenoxid-Propylenoxid-Blockcopolymere, ethoxylierte Triglyceride und/oder Silicontenside.

Beispiele für Lipide mit Esterverbindung können ausgewählt sein aus der Gruppe umfassend Glyceride, Öle, hydrierte Öle, halbsynthetische und synthetische Glyceride, feste und halbfeste Wachse, flüssige Wachse und/oder Phosphatide.

Diese Hilfsstoffe (Bindemittel, synthetische Makromoleküle, Füllstoffe, Dispergiermittel, Konservierungsmittel, Antioxidantien, Benetzungsmittel, Tenside und/oder Weichmacher beziehungsweise Plastifizierungsmittel) können das Anhaften von erfindungsgemäßen Bakteriophagen an Mikropartikel unterstützen. Zusätzlich können die Hilfsstoffe das Dispergieren der Mikropartikel in Flüssigkeiten erleichtern und die Freisetzung des Wirkstoffs beziehungsweise Wirkstoffgemisches kontrollieren.

In einer Auführungsform der vorliegenden Erfindung ist das Material der Hülle der Wirkstoftkapsel Dieses Material kann das Anhaften der erfindungsgemäßen Bakteriophagen unterstützen sowie weiterhin die Freisetzung der Wirkstoffe günstig beeinflussen.

In einer Ausführungsform der vorliegenden Erfindung umfasst das System zusätzlich eine Substratoberfläche, wobei weiterhin das zweite zusätzliche Peptid an der Substratoberfläche haftet. Hierdurch wird der Gesamtzustand beschrieben, dass der bifunktionelle Bakteriophage als Haftvermittler zwischen einem Wirkstoffe umfassenden Partikel und einer Substratoberfläche fungiert. Das Material der Substratoberfläche ist ausgewählt aus der Gruppe umfassend Polycarbonat. Solche Substratoberflächen decken wichtige pharmazeutische und anwendungstechnische Einsatzfelder ab.

Ein weiterer Gegenstand der vorliegenden Erfindung ist die Verwendung des Systems gemäß der vorliegenden Erfindung zur Fixierung von Wirkstoffen auf Substraten.

Die vorliegende Erfindung wird nachfolgend anhand der Beispiele 1 bis 6 weiter erläutert.

### Beispiele:

### Beispiel 1: Panning einer kombinatorischen Phage Display Bibliothek (Ph.D.-12™, New England Biolabs) an einem Polyrethan-Substrat.

Polyurethan-Substrat wurde aus Vermischung äquivalenter Mengen Desmophen® 670 BA und Desmodur® N3300 (Bayer MaterialScience AG) hergestellt, die Aushärtung erfolgte über 16 h bei Raumtemperatur. 20 mg des Substrates wurden 10 min in Tris-Buffered Saline (TBS, bestehend aus 50 mmol/l Tris-HCl pH 7,5, 150 mmol/l NaCl) äquilibriert und 60 min mit 4*10¹⁰ pfu (10 µl der original-Bibliothek) in 1 ml TBS bei Raumtemperatur inkubiert. Das Substrat wurde zehnmal mit je 10 ml TBST (TBS plus 0,1 vol.-% Tween-20) gewaschen (mittels kurzem Vortexen, fünfminütiger Rotation plus 5 s Ultraschallbad). Die erste Elution erfolgte im Sauren durch Eintauchen des Substrats in 1 ml 0,1 mol/l Glycin pH 2,5 für 10 s mit anschließender Neutralisation des Substrats in 1 ml 0,1 mol/l Tris-HCl pH 8 für 1 min. Die erste Elutionslösung wurde durch Zugabe von 200 µl 1 mol/l Tris-HCl pH 8 neutralisiert. Die zweite Elution erfolgte im Basischen durch Eintauchen des Substrats in 1 ml 0,1 mol/l Triethylamin pH 11,5 für 1 min mit anschließender Neutralisation des Substrats in 1 ml 0,1 mol/l Tris-HCl pH 7,5 für 1 min. Die zweite Elutionslösung wurde durch Zugabe von 200 µl 1 mol/l Tris-HCl pH 7,5 neutralisiert. Das Substrat wurde anschließend in TBST aufbewahrt, um zeitliche Ablösungseffekte nicht eluierter Phagen zu beobachten.

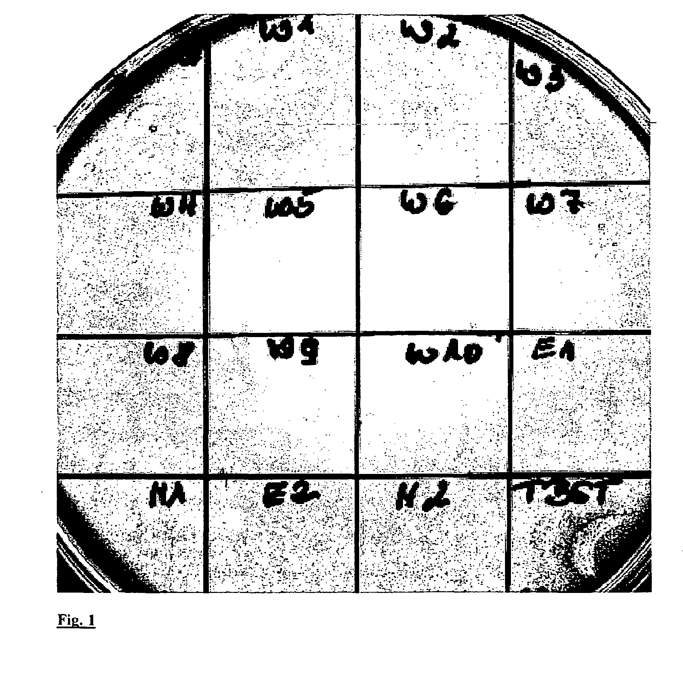

In jeder Fraktion konnte nun qualitativ die Anwesenheit von Phagen mittels eines Plaque-Assay Spot-Tests bestimmt werden (siehe Abbildung 1). Dazu wurden Bakterien des Stamms *E*. *coli* ER2738 (New England Biolabs) auf einer LB-Tet Agarplatte (15 g/l Agar, 10 g/l Bacto-Trypton, 5 g/l Hefeextrakt, 5 g/l NaCl, 20 mg/l Tetracyclin) ausgestrichen und über Nacht bei 37 °C inkubiert. 10 ml LB-Tet Medium (10 g/l Bacto-Trypton, 5 g/l Hefeextrakt, 5 g/l NaCl, 20 mg/l Tetracyclin) wurden mit einer Einzelkolonie ER2738 angeimpft und bis zu einer OD₆₀₀ von 0,4 bei 37 °C geschüttelt. 400 µl davon wurden in 3 ml geschmolzenen LB-Agar-Top (7 g/l Agar, 10 g/l Bacto-Trypton, 5 g/l Hefeextrakt, 5 g/l NaCl) pipettiert und auf einer LB-IPTG/X-gal Platte (15 g/l Agar, 10 g/l Bacto-Trypton, 5 g/l Hefeextrakt, 5 g/l NaCl, 1,25 mg/l Isopropyl-β-D-thiogalactopyranosid, 1 mg/l 5-GBrom-4-chlor-3-indoxyl-β-D-galactopyranosid) ausgebracht. Nach dem Aushärten wurden 3 µl jeder Fraktion auf die Platte aufgetropft und diese über Nacht bei 37 °C inkubiert. Die Anwesenheit von Phagen in jeder Fraktion wurde durch blaue Plaques angezeigt.

Die Elutionsfraktionen (Elution 1 und 2 sowie die beiden Neutralisationslösungen) wurden vereinigt und amplifiziert. Dazu wurden 10 ml LB-Tet Medium mit 100 µl einer Übernachtkultur ER2738 (in LB-Tet) sowie den vereinigten Elutionsfraktionen angeimpft und 4,5 h bei 37 °C geschüttelt. Die Kultur wurde 10 min bei 4500 x g und 4 °C zentrifugiert, und der Überstand anschließend erneut zentrifugiert. Die oberen 4/5 vol. des Überstandes wurden mit 1/5 vol. PEG/NaCl (20 % (w/v) Polyethylenglykol-8000, 2,5 mol/l NaCl) versetzt und 1 h bei 4 °C inkubiert. Die Phagen wurden 20 min bei 16000 x g und 4 °C abzentrifugiert und in 1 ml TBS (50 mM Tris-HCl pH 7,5, 150 mM NaCl) resuspendiert. Die Lösung wird 5 min bei 10000 x g und 4 °C zentrifugiert, der Überstand mit 200 µl PEG/NaCl versetzt und 60 min auf Eis inkubiert. Die phagen wurden 10 min bei 14000 x g und 4 °C abzentrifugiert und in 200 µl TBS resuspendiert.

Der Titer der Phagenlösung wurde durch einen Plaque-Assay bestimmt. Dazu wurden 10 ml LB-Tet Medium mit einer Einzelkolonie ER2738 angeimpft und bis zu einer OD₆₀₀ von 0,4 bei 37 °C geschüttelt. 400 µl davon werden in 3 ml geschmolzenen LB-Agar-Top pipettiert, mit 10 µl einer geeigneten Verdünnung der Phagenlösung versetzt und auf einer LB-IPTG/X-gal Platte verteilt. Nach dem Aushärten wurde die Platte über Nacht bei 37 °C inkubiert. Durch die Anzahl der blauen Plaques ließ sich der Titer der Phagenlösung (in pfu/ml) errechnen.

In den nun folgenden Runden wurde die gesamte Prozedur des Pannings wiederholt, allerdings werden 1-2 x 10¹¹ pfu des amplifizierten Eluats als neue Bibliothek eingesetzt, außerdem wird die Tween-20-Konzentration auf 0,5 % (v/v) erhöht.

Insgesamt wurden drei Panning-Runden durchgeführt. In der resultierenden Population haben sich spezifisch Polyurethan-bindende Phagenklone angereichert, deren Identität durch Sequenzierung des variablen Bereichs ihres Genoms bestimmt werden konnte. Dazu wurden einzelne Phagenklone, z.B. von der LB-IPTG/X-gal-Platte der Titration des Eluats der dritten Runde, mit einer Impföse gepickt und separat amplifiziert: 2 ml LB-Tet Medium wurden mit 100 µl einer Übernachtkultur ER2738 (in LB-Tet) sowie dem Phagenklon angeimpft und 4,5 h bei 37 °C geschüttelt. Die Kultur wurde 10 min bei 4500 x g und 4 °C zentrifugiert, und der Überstand anschließend erneut zentrifugiert. 1 ml der Phagenlösung wurde mit 500 µl PEG/NaCl-Lsg versetzt und 2 h bei 4 °C inkubiert. Die Phagen wurden 15 min bei 14000 x g und 4 °C abzentrifugiert und in 100 µl 10 mM Tris-HCl pH 8,0,1 mM EDTA, 4 M NaI resuspendiert. Die DNS wurde durch 250 µl Ethanol 10 min gefällt und 15 min bei 14000 x g und 20 °C abzentrifugiert. Das Pellet wurde mit 70 %igem Ethanol gewaschen, 1 min bei 14000 x g und 20 °C zentrifugiert, getrocknet und in 30 µl 10 mM Tris-HCl pH 8.0 resuspendiert. Die Sequenzierung der DNS erfolgte mit dem Primer 5'-CCCTCATAGTTAGCGTAACG-3', wobei der Anticodon-Strang der M13-DNS erhalten wird.

### Beispiel 2: erfolgloses Panning einer kombinatorischen Phage Display Bibliothek (Ph.D.-12™, New England Biolabs) an einem Polyurethan-Substrat (ohne Energieeintrag während des Waschens)

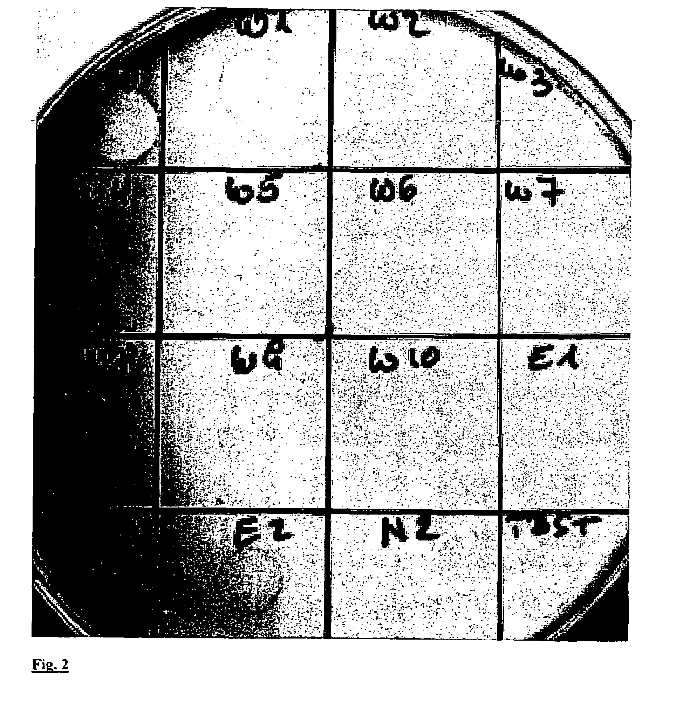

Die Prozedur wird analog zur 1. Runde des Beispiels 1 durchgeführt, außer dass bei den Waschschritten auf gleichzeitigen Energieeintrag verzichtet wurde. Die erhaltenen Fraktionen wurden ebenfalls mittels eines Plaque-Assay Spot-Tests qualitativ auf die Anwesenheit von Phagen hin überprüft (siehe Abbildung 2).

### Beispiel 3: Herstellung einer kombinatorischen gpVIII-Phage Display Bibliothek

Um in das M13-Genom eine Oligonukleotidbank für das Gen VIII (Haupthüllprotein) einzuführen, werden drei Primer benötigt:
1. Bibliotheksoligonukleotid
   5'CGTCACCCTCTGCAGC(NNN)₆AGCAGCGAAAGACACCATGGGAAGC
2. Extension primer I
   5' CGTTCCCATGGTGTCTTTC
3. Extension primer II
   5' ATCGTCACCCTCTGCA

Auf dem Bibliotheksoligonukleotid wird zunächst mit beiden Extensionprimern eine PCR-Reaktion durchgerührt, die zum Erstellen der Bibliothek verwendet wird. Die Bibliothek wird sodann mit NcoI und PstI verdaut und in einen mit NcoI und PstI-Schnittstellen versehenen M13KE-Vektor in die entsprechenden Restriktionsstellen eingefügt.

Um die Bibliothek in das M13 Genom einführen zu können, werden zunächst Restriktionsschnittstellen in die entsprechende Position des Genoms von M13KE eingeführt. Zu Klonierungszwecken werden die Mutationen zunächst auf einem anderen Plasmid (pUC), welches das gpVIII-Procoatgen zwischen den Restriktionsschnittstellen PagI und KpnI beinhaltet, eingebracht (pUC-gp8). Die Einführung der neuen Restriktionsschnittstellen in die gpVIII-Procoatsequenz erfolgt mittels ortsgerichteter Mutagenese (z.B. Sambrook und Russell (Hrsg), 2001, Molecular cloning: A laboratory manual (third edition), Cold Spring Harbor Press, Seite 8.42 bis 8.45) durch Verwendung der folgenden Oligonukleotide:
Mutagene Oligonukleotide
   Nco-A 5'-CGGCGTTCCCATGGTGTCTTTCGCTGC-3'
   Nco-B 5'-GCAGCGAAAGACACCATGGGAACGCCG-3'
   Pst-A 5'-GCTGTCTTTCGCTGCAGAGGGTGACGATCCC-3'
   Pst-B 5'-GGGATCGTCACCCTCTGCAGCGAAAGACAGC-3'

Dadurch wird eine NcoI-Schnittstelle in der Signalsequenz von gpVIII und eine PstI-Schnittstelle in der maturen Region eingefügt (pUC-gp8mut). Diese Mutationen werden anschließend durch Umklonierung aus pUC-gp8mut in die replikative Form (RF) des M13KE-Genoms überführt. Dazu wird pUC-gp8mut mit PagI und KpnI aufgeschnitten und das erhaltene Fragment in den ebenso geschnittenen M13KE-Vektor ligiert. Für die Durchführung von Restriktions- und Ligationsschritten bei doppelsträngigen Plasmiden siehe z.B. Sambrook und Russell (Hrsg), 2001, Molecular cloning: A laboratory manual (third edition), Cold Spring Harbor Press, Seite 1.84 bis 1.87. Für die Durchführung von Transformationen von Plasmiden in E. coli-Bakterien siehe Sambrook und Russell (Hrsg), 2001, Molecular cloning: A laboratory manual (third edition), Cold Spring Harbor Press, Seite 1.105-1.122.

Nach Einführen der Schnittstellen in das Genom von M13KE (M13KE-gp8mut) wird dessen RF-DNA mit den beiden Restriktionsenzymen NcoI und PstI aufgeschnitten und das analog geschnittene Bibliotheks-Oligonukleotid in diesen Vektor ligiert.

Jedes Ligationsprodukt ist in den 6 NNN Positionen unterschiedlich und codiert daher für andere Aminosäuren an diesen 6 Positionen. Die Ligationsansätze stellen die kombinatorische gpVIII-Bibliothek dar und können direkt für das Panning an einem Substrat, analog zu einer kommerziellen gpIII-Phage Display Bibliothek (s.o.), eingesetzt werden.

### Beispiel 4: Herstellung von wirkstoffhaltigen Partikeln mittels Sprühtrocknung

Zur Herstellung von Wirkstoff enthaltenden Matrixpartikel im Mikromaßstab kam in diesem Beispiel das Verfahren der Sprühtrocknung zur Anwendung. Im Folgenden wird der Herstellprozess zur Verkapselung des Insektizids Deltamethrin in einem wasserunlöslichen Polymer (Polystyrol) als Matrix beschrieben.

Die zu versprühende Dispersion war eine O/W Emulsion, die sich wie folgt zusammensetzte:
O-Phase: 74% Dichlormethan, 18% Polystyrol, 8% Deltamethrin
W-Phase: 99% destilliertes Wasser, 1% Alkylpolyglucosid

Zunächst wurde das Deltamethrin und Polystyrol in Dichlormethan gelöst, bevor diese O-Phase in die W-Phase bestehend aus destilliertem Wasser und Alkylpolyglucosid (Emulgator) mit Hilfe eines Ultra Turrax des Typs IKA T18 mit der Stufe 5 für ca. 3 min emulgiert wurde. Die Herstellung der Mikropartikel aus der O/W Emulsion erfolgte mit Hilfe eines Sprühtrockners B-290 der Fa. Büchi. Hierzu wurde die O/W Emulsion mittels einer Schlauchpumpe (2 g/min) zu einer Zweistoffdüse (Düsenkappe 1,5 mm) gefördert und dort mit einem Zerstäubungsgas (Stickstoff) 500 L/h versprüht. Im Sprühzylinder wurden mit Hilfe eines Trocknungsgases (Stickstoff, Gasdurchsatz ca. 31m³/h) die Tröpfchen zu Feststoffpartikeln getrocknet, wobei die Eingangs- und Ausgangstemperatur 100°C und 54°C betrugen. Anschließend wurden in einem Zyklon die Feststoffpartikel vom Trocknungsgas abgetrennt. Die so erhaltenen Mikropartikel bestanden aus Deltamethrin, Polystyrol, Alkylpolyglucosid und einem Restgehalt an Lösemittel (<5 Gew.-%). Die mittels Laserbeugung in wässriger Dispersion bestimmte Partikelgrößenverteilung der wirkstoffhaltigen Partikel zeigt einen d90-Wert der volumengewichteten Verteilung von 28 µm.

### Beispiel 5: Panning einer kombinatorischen Phage-Display Bibliothek an wirkstoffhaltigen Partikeln

20 mg der wirkstoffhaltigen Partikel aus Beispiel 4 wurden 10 min in TBS äquilibriert und 60 min mit 4*10¹⁰ pfu der kombinatorischen gpVIII-Bibliothek aus Beispiel 3 in 1 ml TBS bei Raumtemperatur inkubiert. Die wirkstoffhaltigen Partikel wurden zehnmal mit je 10 ml TBST (TBS plus 0,1 vol.-% Tween-20) gewaschen, indem sie kurz gevortext wurden, 5 min auf einem Rotator und 5 s in einem Ultraschallbad inkubiert wurden. Anschließend wurden sie für 1 min bei 14000 x g abzentrifugiert, der Überstand verworfen und das Pellet in neuem Waschpuffer unter kurzem Vortexen aufgenommen. Die erste Elution erfolgte im Sauren durch Inkubation der wirkstoffhaltigen Partikel in 1 ml 0,1 mol/l Glycin pH 2,5 für 10 s mit anschließender Zentrifugation (1 min bei 14000 x g) und Neutralisation der wirkstoffhaltigen Partikel in 1 ml 0,1 mol/l Tris-HCl pH 8 für 1 min, gefolgt von einer erneuten Zentrifugation. Die erste Elutionslösung wurde durch Zugabe von 200 µl 1 mol/l Tris-HCl pH 8 neutralisiert. Die zweite Elution erfolgte im Basischen durch Inkubation der wirkstoffhaltigen Partikel in 1 ml 0,1 mol/l Triethylamin pH 11,5 für 1 min mit anschließender Zentrifugation (1 min bei 14000 x g) und Neutralisation der wirkstoffhaltigen Partikel in 1 ml 0,1 mol/l Tris-HCl pH 7,5 für 1 min, gefolgt von einer erneuten Zentrifugation. Die zweite Elutionslösung wurde durch Zugabe von 200 µl 1 mol/l Tris-HCl pH 7,5 neutralisiert. Die wirkstoffhaltigen Partikel wurden anschließend in TBST aufbewahrt, um zeitliche Ablösungseffekte nicht eluierter Phagen zu beobachten.

In jeder Fraktion konnte nun qualitativ die Anwesenheit von Phagen mittels eines Plaque-Assay Spot-Tests bestimmt werden. Dazu wurden Bakterien des Stamms *E coli* ER2738 (New England Biolabs) auf einer LB-Tet Agarplatte ausgestrichen und über Nacht bei 37 °C inkubiert. 10 ml LB-Tet Medium wurden mit einer Einzelkolonie ER2738 angeimpft und bis zu einer OD₆₀₀ von 0,4 bei 37 °C geschüttelt. 400 µl davon wurden in 3 ml geschmolzenen LB-Agar-Top pipettiert und auf einer LB-IPTG/X-gal Platte ausgebracht. Nach dem Aushärten wurden 3 µl jeder Fraktion auf die Platte aufgetropft und diese über Nacht bei 37 °C inkubiert. Die Anwesenheit von Phagen in jeder Fraktion wurde durch blaue Plaques angezeigt.

Die Elutionsfraktionen (Elution 1 und 2 sowie die beiden Neutralisationslösungen) wurden vereinigt und amplifiziert. Dazu wurden 10 ml LB-Tet Medium mit 100 µl einer Übernachtkultur ER2738 (in LB-Tet) sowie den vereinigten Elutionsfraktionen angeimpft und 4,5 h bei 37 °C geschüttelt. Die Kultur wurde 10 min bei 4500 x g und 4 °C zentrifugiert, und der Überstand anschließend erneut zentrifugiert. Die oberen 4/5 vol. des Überstandes wurden mit 1/5 vol. PEG/NaCl versetzt und 1 h bei 4 °C inkubiert. Die Phagen wurden 20 min bei 16000 x g und 4 °C abzentrifugiert und in 1 ml TBS resuspendiert. Die Lösung wird 5 min bei 10000 x g und 4 °C zentrifugiert, der Überstand mit 200 µl PEG/NaCl versetzt und 60 min auf Eis inkubiert. Die Phagen wurden 10 min bei 14000 x g und 4 °C abzentrifugiert und in 200 µl TBS resuspendiert.

Der Titer der Phagenlösung wurde durch einen Plaque-Assay bestimmt. Dazu wurden 10 ml LB-Tet Medium mit einer Einzelkolonie ER2738 angeimpft und bis zu einer OD₆₀₀ von 0,4 bei 37 °C geschüttelt. 400 µl davon werden in 3 ml geschmolzenen LB-Agar-Top pipettiert, mit 10 µl einer geeigneten Verdünnung der Phagenlösung versetzt und auf einer LB-IPTG/X-gal Platte verteilt. Nach dem Aushärten wurde die Platte über Nacht bei 37 °C inkubiert. Durch die Anzahl der blauen Plaques ließ sich der Titer der Phagenlösung (in pfu/ml) errechnen.

In den nun folgenden Runden wurde die gesamte Prozedur des Pannings wiederholt, allerdings werden 1-2 x 10¹¹ pfu des amplifizierten Eluats als neue Bibliothek eingesetzt, außerdem wird die Tween-20-Konzentration auf 0,5 % (v/v) erhöht.

Insgesamt wurden drei Panning-Runden durchgeführt. In der resultierenden Population haben sich Phagenklone angereichert, die spezifisch an die wirkstoffhaltigen Partikel binden. Deren Identität konnte durch Sequenzierung des variablen Bereichs ihres Genoms bestimmt werden. Dazu wurden einzelne Phagenklone, z.B. von der LB-IPTG/X-gal-Platte der Titration des Eluats der dritten Runde, mit einer Impföse gepickt und separat amplifiziert: 2 ml LB-Tet Medium wurden mit 100 µl einer Übernachtkultur ER2738 (in LB-Tet) sowie dem Phagenklon angeimpft und 4,5 h bei 37 °C geschüttelt. Die Kultur wurde 10 min bei 4500 x g und 4 °C zentrifugiert, und der Überstand anschließend erneut zentrifugiert. 1 ml der Phagenlösung wurde mit 500 µl PEG/NaCl-Lsg versetzt und 2 h bei 4 °C inkubiert. Die Phagen wurden 15 min bei 14000 x g und 4 °C abzentrifugiert und in 100 µl 10 mM Tris-HCl pH 8,0, 1 mM EDTA, 4 M NaI resuspendiert. Die DNS wurde durch 250 µl Ethanol 10 min gefällt und 15 min bei 14000 x g und 20 °C abzentrifugiert. Das Pellet wurde mit 70 %igem Ethanol gewaschen, 1 min bei 14000 x g und 20 °C zentrifugiert, getrocknet und in 30 µl 10 mM Tris-HCl pH 8.0 resuspendiert. Die Sequenzierung der DNS erfolgte mit dem Primer 5'-CCCTCATAGTTAGCGTAACG-3', wobei der Anticodon-Strang der M13-DNS erhalten wird.

### Beispiel 6: Herstellung eines bifunktionellen Bakteriophagen der ein an gpIII fusioniertes 12 Aminosäuren langes spezifisch an Polycarbonat bindendes Peptid sowie ein an gpVIII fusioniertes, 6 Aminosäuren langes, spezifisch an Polyurethan bindendes Peptid besitzt

Durch das Panning einer kommerziellen Phagenbibliothek (Ph.-D.-12™, New England Biolabs) an Polycarbonat lag in einem Phagenklon ("PC02-11") ein an gpIII fusioniertes, 12 Aminosäuren langes Peptid (ISSKPTSQLTTP) vor, das Polycarbonat-Bindungseigenschaften besitzt. Um die Bifunktionalität zu erreichen, wurde an das gpVIII-Protein desselben Phagenklons ein weiteres, 6 Aminosäuren langes Peptid fusioniert (STTRLR), das Polyurethan-Bindungseigenschaften besitzt. Der Klon mit dieser Sequenz ("PUR01-1") wurde durch das Panning einer randomisierten gpVIII-Phage Display-Bibliothek (s.o.) an Polyurethan gefunden.

Um die Kombination zu erreichen, wurde zunächst die replikative Form von PUR01-1 mit den Restriktionsenzymen PagI und KpnI geschnitten. Das Insert, das das gpVIII-Gen mit fusioniertem Polyurethan-bindenden Peptid enthält, wurde in die ebenfalls mit PagI und KpnI geschnittene replikative Form von PC02-11 ligiert. Durch Transformation in kompetente *E. coli* ER2738-Bakterien (New England Biolabs) wurden bifunktionelle Phagen erzeugt, die an ihren gpIII- und an ihren gpVIII-Proteinen unterschiedliche haftvermittelnde Peptidsequenzen präsentieren.

### Abbildungen:

Abbildung 1: Plaque-Assay von Panning-Fraktionen (Runde 1), unverdünnt aufgetropft auf einen mit E. coli ER2738 versetzten LB-Agar-Top auf einer LB-IPTG/X-gal-Platte. 0: Bibliothek von Runde 1 nach der Substrat-Inkubation; W1.10:Waschfrakionen 1-10 (Benutzung von Ultraschall); E1: Elution 1 (sauer); N1: Neutralisation 1; E2: Elution 2 (basisch); N2: Neutralisation 2; TBST: Aufbewahrung des Substratstücks nach dem Panning in TBST.
Abbildung 2: Plaque-Assay von Panning-Fraktionen (Runde 1), unverdünnt aufgetropft auf einen mit E. coli ER2738 versetzten LB-Agar-Top auf einer LB-IPTG/X-gal-Platte.
0: Bibliothek von Runde 1 nach der Substrat-Inkubation; W1-10:Waschhawonen 1-10 (ohne Benutzung von Ultraschall); E1: Elution 1 (sauer); N1: Neutralisation 1; E2: Elution 2 (basisch); N2: Neutralisation 2; TBST: Aufbewahrung des Substratstücks nach dem Panning in TBST.

## Patentansprüche

1. System, umfassend Wirkstoffe umfassende Partikel und Bakteriophagen vom Typ M 13, wobei das Protein gpIII des Bacteriophagen N-terminal mit einem Peptid der Sequenz ISSKPTSQLTTP fusioniert ist und das Protein gpVIII des Bacteriophagen N-terminal mit einem Peptid der Sequenz STTRLR fusioniert ist, das Peptid mit der Sequenz STTRLR an der Oberfläche des Partikels haftet und das Peptid mit der Sequenz ISSKPTSQLTTP die Haftung des Phagen und damit auch des Wirkstoffe umfassenden Partikels an einer Substratoberfläche ermöglicht, **dadurch gekennzeichnet, dass** das Material der Substratoberfläche Polycarbonat ist, wobei die Wirkstoffe umfassenden Partikel verkapselte Wirkstoffe sind, wobei das Material der Hülle der Wirkstoffkapsel ein Polyurethan ist und wobei die Wirkstoffe ausgewählt sind aus der Gruppe umfassend
1. Insektizide und/oder Fungizide, vorzugsweise Imidacloprid, Deltamethrin, Permethrin, Clotrimazol, Bifonazol, Preventol und/oder Trifloxystrobin; sowie weiterhin
2. Dexpanthenol.

2. Verwendung des Systems nach Anspruch 1 zur Fixierung von Wirkstoffen auf Substraten.

## Claims

1. System, comprising M13-type bacteriophages and particles comprising active substances, where the gpIII protein of the bacteriophage is N-terminally fused to a peptide of sequence ISSKPTSQLTTP and the gpVIII protein of the bacteriophage is N-terminally fused to a peptide of sequence STTRLR, where the peptide of sequence STTRLR adheres to the surface of the particle and where the peptide of sequence ISSKPTSQLTTP makes possible the adhering of the phage, and hence also the particle comprising active substances, to a substrate surface, **characterized in that** the material of the substrate surface is polycarbonate, where the particles comprising active substances are encapsulated active substances, where the material of the shell of the active substance capsule is a polyurethane and where the active substances are selected from the group comprising
1. insecticides and/or fungicides, preferably imidacloprid, deltamethrin, permethrin, clotrimazole, bifonazole, preventol and/or trifloxystrobin; and furthermore
2. dexpanthenol.

2. Use of the system according to Claim 1 for fixing active substances to substrates.

## Revendications

1. Système comprenant des particules, 1 comprenant des substances actives, et des bactériophages du type M13, la protéine gpIII du bactériophage étant fusionnée à l'extrémité N-terminale avec un peptide ayant la séquence ISSKPTSQLTTP et la protéine gpVIII du bactériophage étant fusionnée à l'extrémité N-terminale avec un peptide ayant la séquence STTRLR, le peptide ayant la séquence STTRLR adhérant la surface de la particule et le peptide ayant la séquence ISSKPTSQLTTP permettant l'adhérence du phage et donc également de la particule comprenant des substances actives à une surface de support, **caractérisé en ce que** le matériau de la surface de support est du polycarbonate, les particules comprenant des substances actives étant des substances actives encapsulées, le matériau de l'enveloppe des capsules de substances actives étant un polyuréthane et les substances actives étant choisies dans le groupe comprenant
1. des insecticides et/ou des fongicides, de préférence l'imidaclopride, la deltaméthrine, la perméthrine, le clotrimazole, le bifonazole, le Preventol et/ou la trifloxystrobine, ainsi qu'en outre
2. du dexpanthénol.

2. Utilisation du système selon la revendication 1, pour la fixation de substances actives à des supports.
